# EUROPEAN PATENT APPLICATION

(11) **EP 4 067 477 A1**
(43) Date of publication of application: **05.10.2022**
(21) Application number: 20892125.4
(22) Date of filing: 11.06.2020
(51) Int. Cl.: C12N 5/00, C12M 1/42, A61L 27/38, A61L 27/20

(54) **CELL SHEET COMPRISING HYALURONIC ACID AND POLYETHYLENE GLYCOL, AND METHOD FOR PRODUCING SAME**

(30) Priority: 25.11.2019 KR 20190152197
(71) Applicant: Akrocell Bioscience, Inc., Dongdaemun-gu, Seoul 02455 (KR)
(72) Inventor: LEE, Eunah, Seoul 05262 (KR); OH, Dong In, Seoul 01727 (KR)
(74) Representative: Klunker IP Patentanwälte PartG mbB
(86) International application number: PCT/KR2020/007604
(87) International publication number: WO 2021/107303

(57) **Abstract**

Disclosed is a method of constructing a cell sheet using only cells without a support. More particularly, a method of manufacturing a multi-layered cell sheet without a separate lamination step and a cell sheet manufactured by the method are disclosed.

## Description

### [Cross-Reference to Related Application]

This application is a National Stage Entry of PCT International Application No. PCT/KR2020/007604, which was filed on June 11, 2020, and which claims priority to Korean Patent Application No. 10-2019-0152197, filed on November 25, 2019 in the Korean Intellectual Property Office, the disclosure of which is incorporated herein by reference.

### [Technical Field]

The present invention relates to a method of constructing a cell sheet in a non-adherent state without a support, and more particularly to a method of manufacturing a multi-layered cell sheet without a separate lamination step and to a cell sheet manufactured by the method.

### [Background Art]

Since cells exhibit higher activity when they maintain an adherent state, a cell sheet or scaffold-based cell complex, in which cell-cell or cell-matrix bonding is maintained, rather than a suspension collected in the form of single cells is advantageous in maximizing the activity of transplanted cells.

For this reason, various techniques for forming implantable cell sheets have been developed. Cells cultured in such a sheet state have an advantage in that the activity thereof can be maintained high by maintaining cell-cell contact and pericellular matrix molecules.

However, a cell structure cultured in a single-layer sheet state has a disadvantage that structural damage thereof may easily occur in a process of handling for cell transplantation or the like. In addition, when cell sheets are multi-layered to have high integrity so as to secure the thickness of the tissue to be regenerated, there is a problem that an additional technology is required.

Cell therapy in the form of injecting or transplanting cells itself is being mainly used until now, but with the increase in demand for tissue engineered preparations manufactured by culturing transplantable tissues in vitro, continuous development therefor is required.

### [Disclosure]

### [Technical Problem]

Therefore, the present invention has been made in view of the above problems, and it is one object of the present invention to provide a method of manufacturing a plate-shaped cell sheet, the method including a step of treating with hyaluronic acid (HA) and polyethylene glycol (PEG).

It is another object of the present invention to provide a plate-shaped cell sheet manufactured by the method.

It is yet another object of the present invention to provide a graft material for cartilage repair including the plate-shaped cell sheet and a biodegradable polymer material.

### [Technical Solution]

In accordance with an aspect of the present invention, the above and other objects can be accomplished by the provision of a method of manufacturing a cell sheet, the method including: a) a step of sedimenting and culturing cells in a growth medium including hyaluronic acid; and b) a step of adding a growth medium including polyethylene glycol.

Specifically, the cell sheet manufactured by the method may have a plate shape.

In the present invention, the "cell sheet" refers to one or more type of cells arranged in a layered structure, and may have a plate shape, without being limited thereto. The term "multi-layered cell sheet" refers to a set of cell sheets formed by stacking one or more cell sheets in a vertical direction. Hereinafter, the "cell sheet" as used herein is used to refer to both a single-layered cell sheet and a multi-layered cell sheet.

In the present invention, the "laminated" or "formed" means that thin film layers are stacked layer by layer in the order of creation. The laminated structure is not specifically limited as long as it is manufactured by a method known in the art.

In the present invention, the "hyaluronic acid (HA)", which is one of complex polysaccharides composed of amino acids and uronic acid, is a high molecular compound composed of N-acetylglucosamine and glucuronic acid. Hyaluronic acid is mainly present in the vitreous body of the eye or the umbilical cord, and plays a role in preventing the penetration of bacteria or poisons. In addition, hyaluronic acid, which is a drug used for purposes such as an adjuvant for various ophthalmic surgeries, intra-articular injections, artificial tears, and wound healing, is a type of polysaccharides that can contain water in 300 to 1000 times its own weight and is known for its excellent moisturizing properties. However, there has been no report on a method for preparing a cell sheet by using hyaluronic acid together with polyethylene glycol (PEG) as in the present invention.

"PEG," which is a kind of surfactant, is a chemical ingredient that is used in various products such as lotions, creams, shampoos, etc. by stably maintaining products during cosmetic manufacturing and performing a role like glycerin. However, there has been no report on a method for preparing a cell sheet by using PEG together with HA as in the present invention.

Specifically, cells used in the cell sheet may be one or more types selected from the group consisting of epidermal cells, fibroblasts, hepatocytes, mesenchymal stem cells, and chondrocytes, and more specifically, may be chondrocytes, without being limited thereto.

In an embodiment of the present invention, the chondrocytes are derived from rabbit meniscus, and a plate-shaped cell sheet was prepared using the chondrocytes, without being limited thereto.

Cells in the cell sheet may be differentiated from human pluripotent stem cells.

In the present invention, "human pluripotent stem cells (PSCs)" refer to stem cells with the ability to differentiate into all three germ layers, i.e., endoderm, mesoderm, and ectoderm, constituting the human body. Human PSCs are evaluated as very useful materials for clinical trials for the treatment of various diseases, new drug development, toxicity evaluation, disease modeling and early embryogenesis research.

Specifically, the cell sheet may be characterized in that aggregation of cells is suppressed.

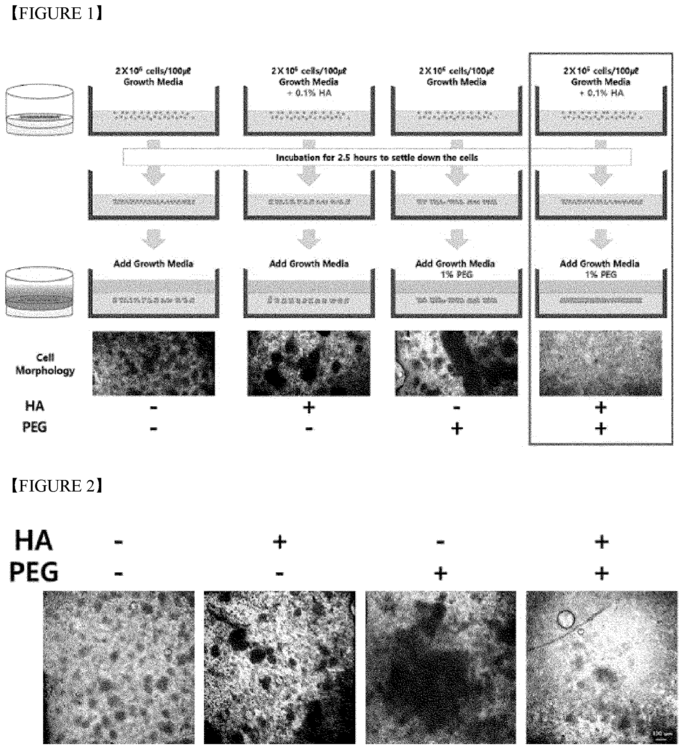

In an embodiment of the present invention, the cell sheet is characterized by being formed without a separate support (scaffold) in a non-adherent state . It was confirmed that the formed cell sheet hardly exhibits spheroids due to significantly inhibited cell aggregation, and cells maintain a sheet formin the form of being sedimented to the bottom in a culture medium(Experimental Example 1, FIGS. 2 to 4).

Such a result is because the behavior of cells is controlled by the differential actions of hyaluronic acid (HA) and polyethylene glycol (PEG) present in a culture medium so that the cell behavior is changed not to form spheroids. In addition, it seems that, since polyethylene glycol (PEG) contained in a medium exhibits repulsive force against cells whereas hyaluronic acid (HA), which has the properties of a hydrogel, maintains cell-to-cell bonding while maintaining homogenous dispersion within the medium components, the force to minimize the interface between the PEG and the cells contained in the medium plays a role in maintaining the shape of the plate-like cell slab.

In the present invention, the "hydrogel" is a material that can contain a large amount of water, and is a material or form of being capable of easily transmitting and moving substances necessary for cell survival such as oxygen, water, water-soluble nutrients, enzymes, and polypeptides such as cytokines, and waste products. In general, "hydrogel" is biocompatible. The shape or form of the hydrogel is not specifically limited as long as it can be incorporated into a cell sheet, but, for example, various shapes or forms such as fine particles, a granular form, a film form, a tubular form, a disk form, a network form, a mesh form, a porous form, a suspended state or a dispersed form may be used. Among them, hydrogel particles obtained by solidifying an aqueous solution containing colloidal particles are preferable. The particles may be formed of any materials so long as the above properties of hydrogel are exhibited. For example, the particles may be formed of a water-soluble, hydrophilic, or water-absorbing synthetic polymer such as polyacrylamide, polyacrylic acid, polyhydroxyethyl methacrylate, polyvinyl alcohol, polylactic acid, polyglycolic acid or polyethylene glycol; and a hydrogel obtained by chemically crosslinking a polysaccharide, a protein, nucleic acid, or the like. Examples of a polysaccharide include glycosaminoglycan such as hyaluronic acid and chondroitin sulfate, starch, glycogen, agar, pectin, fiber, and the like, without being limited thereto. In addition, examples of protein include collagen and gelatin as a hydrolyzate thereof, proteoglycan, fibronectin, vitronectin, laminin, entactin, tenascin, thrombospondin, von Willebrand factor, osteopontin, fibrinogen, and the like, without being limited thereto. Particularly, particles made of a material that is biocompatible and degraded by cells in the living body are suitable for the present invention. Hyaluronic acid and polyethylene glycol are most preferred.

Specifically, the cell sheet may be composed of multi-layered cells, and each cell layer constituting the multi-layer may be characterized by having structural continuity while maintaining the integrity of cell layers.

Existing cell sheets are usually formed based on a support. In the case of support-based cell sheets, it is difficult to uniformly distribute cells inside the support, and the cell density is lowered by the volume occupied by the support, so that there is a problem that the number of cells transferred during transplantation is low. In addition, there is a problem that structural deformation and changes in tissue continuity may occur as a remaining support is gradually decomposed.

Meanwhile, despite the above problems, a cell sheet composed of a single layer of cells through 2D culture without a support has the disadvantage that it is easily torn and it is difficult to handle due to weak strength thereof. In addition, since the existing cell sheet is composed of a single layer of cells, there is a limitation in that it is difficult to deliver high concentrations of cells when used for transplantation. To solve these problems, attempts have been made to form a multilayer structure to increase cell density, but there are limitations in that the process of forming the multilayer structure is complicated and it is difficult to implement 100% interlayer integrity.

When a multilayered cell structure is formed by seeding cells at a high concentration, a high number of cells can be delivered per unit area, but the cells seeded at a high concentration are locally aggregated to form spheroids. Accordingly, there is a limitation that a cell sheet having a uniform thickness and structural continuity cannot be formed.

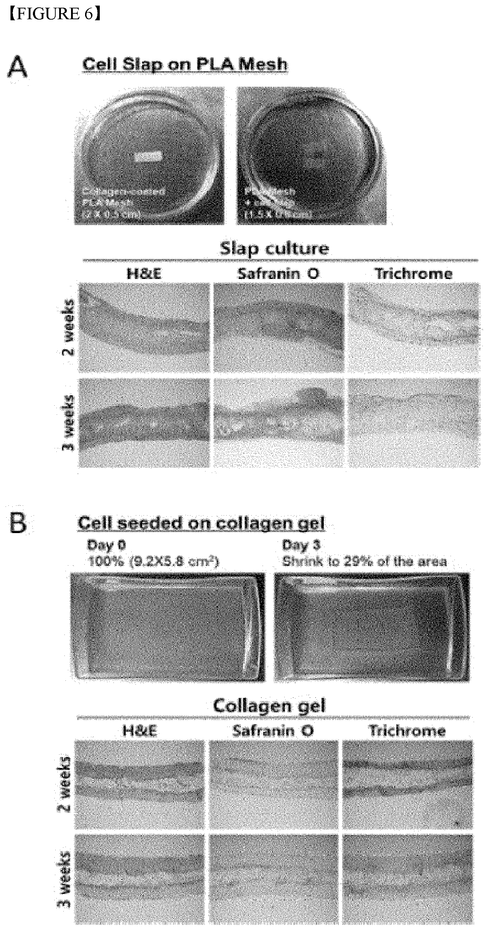

On the other hand, it was confirmed that the cell sheet of the present invention has a high cell transfer efficiency by forming a high-density sheet composed of multi-layered cells without a support and is advantageous for tissue regeneration (Experimental Example 4 and FIG. 6). In addition, since a support is unnecessary in the process of forming the cell sheet, there is no concern about structural deformation occurring during the decomposition of the support. Further, since the behavior of cells is controlled through the composition of a medium instead of coating, there is an advantage that it is easy to apply the present invention because it is the same as existing processes of dealing with cells, without adding other processes.

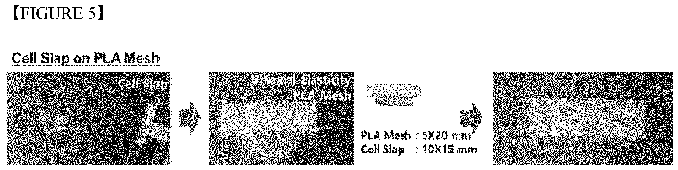

Since the formation of the cell sheet of the present invention is induced under a non-adherent condition, the cell-substrate interaction is not significantly limited. In addition, since the surface treatment of a culture container is unnecessary, there is an advantage that it is possible to construct a culture vessel of various materials using 3D printing or the like. Further, since the cell sheet of the present invention can form a cell structure composed of multi-layered cells without a lamination process, structural stability is increased because the process does not depends on layering procedure. In addition, it was confirmed that the lamination process can be omitted, thereby shortening the process and producing a thick layer of cells by itself, enabling more stable handling (Experimental Example 3 and FIG. 5).

In accordance with another aspect of the present invention, there is provided a cell sheet manufactured by the method.

In accordance with yet another aspect of the present invention, there is provided a graft material for tissue repair, including the cell sheet.

Specifically, the tissue type may be one or more selected from the group consisting of cartilage, bone, cornea, conjunctiva, adipose tissue, skin, muscle, fascia, tendon, aponeurosis, ligament, joint capsule, bursa, and epithelial tissue, more specifically cartilage, without being limited thereto.

Cells used in the cell sheet may be cells differentiated from human pluripotent stem cells into cells of a desired tissue.

In the present invention, the "tissue repair" may be used as a concept including both regeneration and healing of tissues, "regeneration" refers to reconstitution of a damaged area with the same tissue composition as damaged cells, and "healing" refers to a fibroproliferative response that patches a damaged tissue.

In the present invention, "for tissue repair" is not limited to a specific use so long as it is used to repair damaged tissue, and may include not only cartilage damage, muscle or tendon damage, but also cosmetic filler use, cosmetic implant use.

Since cartilage and soft tissue do not regenerate or heal themselves after being damaged, it is inappropriate to apply a method for promoting tissue growth thereto, unlike other tissues, and it is known that a treatment through transplantation is effective therefor.

The "graft material for tissue repair" of the present invention refers to a composition that treats damage to a defective or depressed area and restores the original volume thereof by filling a damaged area using physical properties when tissues, such as cartilage, bone, cornea, conjunctiva, adipose tissue, skin, muscle, fascia, tendon, aponeurosis, ligament, joint capsule, and bursa, are damaged. The graft material for tissue repair may be appropriately modified and applied without being specifically limited so long as it is used for the purpose.

It was confirmed that the cell sheet of the present invention is composed of cells at a high concentration without including a separate support and thus has excellent therapeutic and regenerative effects on a transplanted tissue site (FIG. 6). Accordingly, a graft material for tissue repair including the cell sheet can exhibit high tissue regeneration efficiency.

### [Advantageous effects]

By a method of manufacturing a cell sheet of the present invention, a cell sheet can be formed in a non-adherent state without a support and can be detached and handled without a separate coating treatment. In addition, by the method of the present invention, a cell sheet implanted with a high number of cells per unit area without going through a separate lamination process or multilayer process can be provided.

It should be understood that the effects of the present invention are not limited to the effects described above, but include all effects that can be deduced from the detailed description of the present invention or the constitution of the invention described in the claims.

### [Description of Drawings]

FIG. 1 schematically illustrates a method of manufacturing a cell sheet using hyaluronic acid (HA) and polyethylene glycol (PEG).
FIG. 2 illustrates cell aggregation degrees of cell sheets manufactured by methods of Example 1 and Comparative Examples 1 to 3 after culturing for 24 hours.
FIG. 3 illustrates the number of nodules observed in each of Example 1 and Comparative Examples 1 to 3.
FIG. 4 illustrates the size of nodules observed in each of Example 1 and Comparative Examples 1 to 3.
FIG. 5 illustrates a process of attaching a cell sheet of Example 1 to a Poly Lactic Acid (PLA) mesh.
FIG. 6 illustrates the shape of tissue formed after attaching a cell sheet to a PLA mesh in the form of a sandwich, and then inducing differentiation into cartilage for 2 or 3 weeks; and a matrix observed through histological staining.

### [Best Mode]

Hereinafter, the present invention is described in detail with reference to examples. However, the following examples are only for aid in understanding of the present disclosure, and the present invention is not limited to the following examples.

### Example 1. Method of manufacturing cell sheet including HA and PEG

A method of manufacturing a cell sheet using hyaluronic acid (HA) and polyethylene glycol (PEG) is briefly illustrated in FIG. 1.

Specifically, a bottom surface of a rectangular well whose all sides were closed was coated with poly-HEMA (poly(2-hydroxyethyl methacrylate) to prevent cell binding.

In a state where binding of cells to the bottom surface of the well is prevented, chondrocytes derived from rabbit meniscus between 3 and 5 passages were seeded in a growth medium containing 1 mg/ml hyaluronic acid (HA) at a concentration of 2 × 10⁶/100 µl and cultured for 2.5 hours to induce a cell layer to uniformly sink. Here, the growth medium was prepared by adding 5% fetal bovine serum (FBS, Lonza), 1% penicillin/streptomycin (Welgene), 2mM L-glutamine (Welgene), 10⁻⁸M dexamethasone (Sigma Aldrich) and 10⁻⁴M ascorbic acid (Sigma Aldrich) to alpha-Minimum Essential Medium (a-MEM, Gibco Invitrogen).

Next, 1% polyethylene glycol (PEG) was added to the growth medium and cells were additionally cultured therein. As a result, a cell sheet of Example 1 was produced.

### Comparative Example 1. Production of cell sheet treated with only growth medium

A cell sheet of Comparative Example 1 was manufactured under the same conditions as in Example 1 except that hyaluronic acid (HA) and polyethylene glycol (PEG) were not added.

### Comparative Example 2. Production of cell sheet treated with HA only

A cell sheet of Comparative Example 2 was manufactured in the same manner as in Example 1 using hyaluronic acid (HA) containing a growth medium, except that a growth medium excluding polyethylene glycol (PEG) was added after culturing for 2.5 hours.

### Comparative Example 3. Production of cell sheet treated with PEG only

A cell sheet of Comparative Example 3 was manufactured under the same conditions as in Example 1, except that a growth medium excluding hyaluronic acid (HA) was used when initially seeding chondrocytes derived from rabbit meniscus.

The manufacturing methods of Example 1 and Comparative Examples 1 to 3 are briefly illustrated in FIG. 1.

### Experimental Example 1. Analysis of cell sheet shapes dependent upon presence or absence of HA and PEG

To analyze whether the shape of the cell sheet depends upon the presence or absence of hyaluronic acid (HA) and polyethylene glycol (PEG), it was confirmed whether the cell sheets manufactured by the above methods had a uniform surface thickness without cell aggregation.

Specifically, the degree of cell aggregation was checked 24 hours after maintaining the culture state for the cell sheets manufactured by the methods of Example 1 and Comparative Examples 1 to 3. Results are illustrated in FIG. 2.

As a result, it was confirmed that whether hyaluronic acid (HA) at a concentration of 1 mg/ml was included in the initial cell seeding and whether 1% PEG was included in a medium when the medium was added greatly affected the degree of aggregation of cells, as shown in FIG. 2.

Specifically, in the case of Comparative Example 1 in which both HA and PEG were excluded, a large number of spheroids of low density were formed after 24 hours of culture similarly to general spheroid formation conditions. In the case of Comparative Example 2 containing HA at the time of cell seeding, the tendency to form relatively dense spheroids was high. In the case of Comparative Example 3 using a medium containing only PEG without HA, aggregation of cells occurred in a disorderly manner to form huge spheroids.

On the other hand, it was confirmed that in the case of Example 1 wherein cells were seeded under a condition containing HA and a medium containing PEG was added, cell aggregation was minimally suppressed so that spheroids did not appear, and the cells maintained a sheet shape in a state of being sedimented on the bottom in the culture medium and maintained a relatively uniform thickness.

This result seems to be because the spheroid formation tendency induced by cell aggregation was changed by controlling the behavior of cells by the differential action of the two hydrogel components present in the culture medium.

That is, it seems that, while HA having the characteristics of a hydrogel maintains intercellular bonding while evenly distributing the medium components, PEG contained in the medium exhibits repulsive force against cells, so that a force to minimize the interface between PEG and cells contained in the medium is generated, thereby maintaining the shape of the cell sheet.

### Experimental Example 2. Confirmation of nodule formation dependent upon presence or absence of HA and PEG

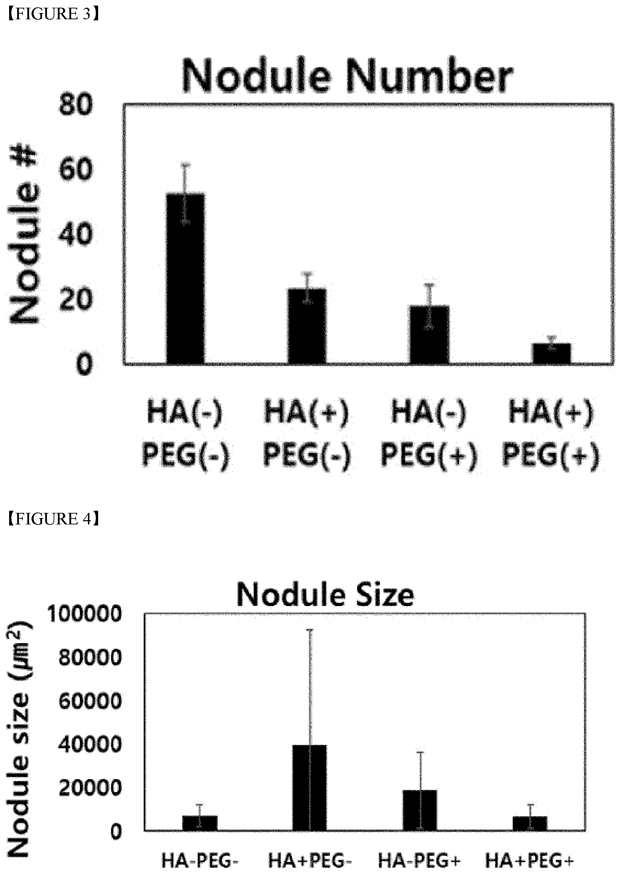

To investigate the degree of nodule formation dependent upon the presence or absence of HA and PEG in the same manner as in Experimental Example 1, the number and sizes of nodules of each of Example 1 and Comparative Examples 1 to 3 were observed. Results of the number of the nodules are illustrated in FIG. 3, and results of the size thereof are illustrated in FIG. 4.

As shown in FIG. 3, it was confirmed that the number of nodules was the highest in the untreated Comparative Example 1, the number of nodules formed in the group treated with only hyaluronic acid (Comparative Example 2) in which relatively large spheroids were formed; and the group treated with only PEG (Comparative Example 3) in which cell aggregation occurred in a disorderly manner was smaller than that of Comparative Example 1, and the number of nodules formed in Example 1 treated with both HA and PEG was the smallest.

In addition, it was confirmed that the sizes of nodules in the untreated Comparative Example 1, and Example 1 treated with both HA and PEG were significantly smaller than those of Comparative Examples 2 and 3, as shown in FIG. 4.

When the above results are taken together, the number of aggregates formed in Example 1 treated with both HA and PEG was the smallest and the size thereof was also the smallest. This result corresponds to a very important content for overcoming the technical limitations existing in the prior art.

Existing single cell layer-based cell sheets have weak physical strength and limitations in that the cell concentration is low when used for transplantation. To overcome the limitations of such a single cell layer-based cell sheet, a cell sheet composed of multi-layered cells and a method of manufacturing the same have been required. However, attempts to form a cell sheet composed of multi-layered cells by seeding cells at a high concentration do not solve the problem because a large number of spheroids are formed due to cell aggregation increasing as the concentration of cells increased. Accordingly, a separate lamination process is required to manufacture a cell sheet composed of multi-layered cells, which causes structural instability and process extension.

However, the problem of excessive spheroid formation can be addressed by using the method for manufacturing a cell sheet containing HA and PEG of the present invention, so that a cell sheet composed of multi-layered cells can be manufactured without a separate lamination process.

### Experimental Example 3. Confirmation of usability of cell sheet including HA and PEG

It was confirmed whether the limitations and inconveniences existing in a process of using a cell sheet manufactured by an existing cell sheet manufacturing method can be overcome by using the cell sheet manufactured in Example 1.

In the case of manufacturing a cell sheet using an existing support, it is difficult to cleanly separate the cell sheet without a separate treatment process, and furthermore, there is a possibility of structural deformation of the cell sheet and a possibility of change in tissue continuity during the disassembly of the support or separation from the support.

In addition, in the case of existing single cell layer-based cell sheets, there is a disadvantage that they are easily torn due to weak strength and it is difficult to handle the sheets without a separate handling tool. In the case of forming a multilayer structure, the process is very complicated, it is difficult to implement 100% interlayer integrity, and it is impossible to form a cell sheet having a uniform thickness and structural continuity.

The cell sheet of the present invention is characterized by being formed under a non-adherent condition without a support, being composed of multi-layered cells without a separate lamination process, and being formed to be sufficiently thick. To confirm such characteristics, a process of attaching the cell sheet of Example 1 to a Poly Lactic Acid (PLA) mesh was demonstrated (FIG. 5).

As shown in FIG. 5, the cell sheet was formed to be wider than the PLA mesh, and then attached around the front and back of a collagen-coated PLA mesh, thereby forming a complex for cartilage differentiation.

Here, since the cell sheet of Example 1 was formed under a non-adherent condition without a separate support, it was possible to separate a uniform cell sheet having a constant thickness only with a cell scraper.

In addition, it was confirmed that since the separated cell sheet was formed to be sufficiently thick with multi-layered cells, it was possible to handle without tearing only with tweezers without a separate handling tool.

That is, it was confirmed that since the cell sheet manufactured by the method of manufacturing a cell sheet of the present invention is formed under a non-adherent condition without a support, the multi-layered cell structure is formed by itself without a separate lamination process, so that the multi-layered cell structure is structurally stable and has uniform thickness and structural continuity.

### Experimental Example 4. Confirmation of matrix formation effect of cell sheet prepared in the presence of HA and PEG

To investigate the matrix-forming effect of the cell sheet manufactured by the method of the present invention, the cell sheet was attached to a PLA mesh in a sandwich form. Whether cells on both surfaces of the mesh were combined with each other and integrated into a single tissue was investigated, and the efficacy of matrix formation during cartilage differentiation was investigated.

Specifically, a cell sheet seeded at a high concentration on a surface of a collagen gel sheet and the cell sheet of Example 1 were respectively attached to the PLA mesh in a sandwich form, and then cartilage differentiation was induced for 2 or 3 weeks. Next, The shapes of the formed tissues and matrix formation were confirmed through histological staining (FIG. 6).

Specifically, the fixed tissue was paraffin-sectioned to a thickness of 5 to 7 µm and dried, and then subjected to hematoxylin/eosin staining (H&E staining), and then the tissue shape was observed with a high-resolution optical microscope. To confirm the formation of a cartilage-specific matrix through Safranin O staining, the nucleus was stained with Weigert's hematoxylin for 5 minutes, and then washed with distilled water for 10 minutes. The tissue was immersed in 70% ethanol solution to adapt to the ethanol solution, and then stained with 0.02% fast green for 5 minutes and washed with 1% acetic acid. After staining with 0.1% aqueous safranin O for 5 minutes, the tissues were dehydrated by sequentially adapting to 70%, 80%, 90%, and 100% alcohol, and then placed in xylene to adapt, and then mounted with a plastic mounting solution (mountant).

To investigate whether the collagen gel sheet remains by staining the fibrous matrix protein by the Trichrome staining method, the nucleus was stained with Weigert's hematoxylin for 10 minutes, and then washed with distilled water for 10 minutes, and the cytoplasm was stained with an aqueous solution of Biebrich scarlet acid red fuchsin for 5 minutes. After staining the fibrous substrate with aniline blue, the tissue was stained and fixed with 1% acetic acid, dehydrated by sequentially adapting to 70%, 80%, 90%, and 100% alcohol, and placed in xylene to adapt, and then mounted with a plastic mounting solution (mountant). All reagents were purchased from Sigma Aldrich unless otherwise indicated.

As a result, as illustrated in FIG. 6A, it was confirmed that the tissues formed from the cell sheet of Example 1 were combined with each other after penetration into the PLA mesh located in the center to form a single tissue, and the formation of the cartilage-specific matrix was excellent.

On the other hand, in the cell sheet formed using the collagen gel sheet, the degree of penetration or migration of cells seeded on the collagen surface into the PLA mesh was remarkably low, so that two separate layers were formed with the PLA mesh as a boundary, as illustrated in FIG. 6B. In addition, compared with the cell sheet of Example 1, it was confirmed that the degree of cartilaginous matrix formation was very low, and the cell-seeded collagen gel sheet continued to remain (trichrome staining result).

From the above results, it was confirmed that the cell sheet of the present invention including HA and PEG was formed without a separate support such as a collagen gel sheet and exhibited high structural continuity with perfect integrity as well as high cell transfer efficiency and advantageous tissue regeneration due to high cell density.

The aforementioned description of the present invention is provided by way of example and those skilled in the art will understand that the present invention can be easily changed or modified into other specified forms without change or modification of the technical spirit or essential characteristics of the present invention. Therefore, it should be understood that the aforementioned examples are only provided by way of example and not provided to limit the present invention. For example, each of constituents described as a single form may be separately implemented and, similarly, constituents described as being separated may be implemented in a combined form.

It should be understood that the scope of the present invention is defined by the following claims and the invention is to cover all modifications, equivalents, and alternatives falling within the spirit and scope of the invention as defined by the claims.

## Claims

1. A method of manufacturing a cell sheet, the method comprising:
a) a step of sedimenting and culturing cells in a growth medium comprising hyaluronic acid; and
b) a step of adding a growth medium comprising polyethylene glycol.

2. The method according to claim 1, wherein the cells are one or more selected from the group consisting of epidermal cells, fibroblasts, hepatocytes, mesodermal stem cells, and chondrocytes.

3. The method according to claim 1, wherein the cell sheet has a plate shape.

4. The method according to claim 1, wherein the cell sheet has suppressed cell aggregation.

5. A cell sheet manufactured according to any one method of claims 1 to 4.

6. A graft material for tissue repair, comprising the cell sheet according to claim 5.

7. The graft material according to claim 6, wherein the tissue type is one or more selected from the group consisting of cartilage, bone, cornea, conjunctiva, adipose tissue, skin, muscle, fascia, tendon, aponeurosis, ligament, joint capsule, bursa, and epithelial tissue.
